# EUROPÄISCHE PATENTSCHRIFT

(11) **EP 3 310 483 B1**
(45) Veröffentlichungstag und Bekanntmachung des Hinweises auf die Patenterteilung: **06.07.2022**
(21) Anmeldenummer: 16760383.6
(22) Anmeldetag: 31.05.2016
(51) Int. Cl.: G01N 33/487, B01L 3/00

(54) **VERFAHREN UND VORRICHTUNG ZUM BEREITSTELLEN VON EINE GETROCKNETE FLÜSSIGKEITSMENGE, INSBESONDERE BLUT, AUFWEISENDEN, SAUGFÄHIGEN PROBENTRÄGERN**
METHOD AND DEVICE FOR PROVIDING ABSORBENT SAMPLE CARRIERS WHICH COMPRISE A DRIED QUANTITY OF LIQUID, IN PARTICULAR BLOOD
PROCÉDÉ ET DISPOSITIF DE PRÉPARATION D'UNE QUANTITÉ DE LIQUIDE SÉCHÉE, NOTAMMENT DU SANG, COMPRENANT DES PORTE-ÉCHANTILLONS ABSORBANTS

(30) Priorität: 17.06.2015 DE 102015007642
(43) Veröffentlichungstag der Anmeldung: 25.04.2018
(73) Patentinhaber: Sarstedt AG & Co. KG, 51588 Nürnbrecht (DE)
(72) Erfinder: WEINSTOCK, Mark, 57612 Helmenzen (DE); WEGENER, Christian, 51588 Nümbrecht (DE)
(74) Vertreter: Grosse, Wolf-Dietrich Rüdiger
(86) Internationale Anmeldenummer: PCT/DE2016/000229
(87) Internationale Veröffentlichungsnummer: WO 2016/202319

(56) Entgegenhaltungen:
- WO-A1-2013/148071
- WO-A1-2015/058958
- WO-A2-2015/044454
- US-A- 4 883 764
- US-A1- 2001 039 057
- US-A1- 2004 126 281
- US-A1- 2013 280 725
- US-A1- 2013 281 316

## Beschreibung

Probenträger-Bereitstellungsschachtel zum Bereitstellen von eine getrocknete Flüssigkeitsmenge, insbesondere Blut, aufweisenden, saugfähigen Probenträgern

Die Erfindung betrifft eine Probenträger-Bereitstellungsschachtel zum Bereitstellen von eine getrocknete Flüssigkeitsmenge, insbesondere Blut, aufweisenden, saugfähigen Probenträgern zu analytischen Auswertungen, wobei eine aufgegebene unbestimmte Flüssigkeitsmenge durch Kapillarwirkung auf einen saugfähigen Probenträger appliziert wird und der Probenträger nach dem Trocknen der Flüssigkeitsmenge an eine weiterverarbeitende Einheit abgegeben wird.

Es ist seit langem für verschiedenartige klinische Untersuchungen an Blutproben, aber auch auf anderen Anwendungsfeldern, wie in der Pharmaforschung, der klinischen Chemie, der therapeutischen Arzneimittelüberwachung oder der forensischen Toxikologie und der Dopinganalytik die sogenannte Dried-Blood-Spot (DBS)-Analyse bekannt, vor allem in Verbindung mit nachweisstarken LC-MS/MS (liquid chromatography tandem mass spectroscopy)-Systemen. Zur DBS-Analyse reichen wenige aus der Fingerbeere oder Ferse eines Patienten entnommene Blutstropfen aus. Das entnommene Blut wird, wie beispielsweise aus der US 8 586 382 B2 bekannt, auf speziellem, saugfähigem Testpapier als Probenträger von Hand in kreisförmig markierte Bereiche aufgetragen, nach der Trocknung des Blutes und ggf. vorheriger Aufbewahrung zur Analyse gegeben, was in einer weiterverarbeitenden Einheit in einem automatisierten Prozessablauf eingebunden werden kann. Da es sich bei dem aufgetragenen, getrockneten Blut um eine unbestimmte Flüssigkeitsmenge handelt, ist es auf jeden Fall vorab erforderlich, aus dem Probenträger bzw. den markierten Bereichen des Testpapiers eine Scheibe von wenigen Millimetern Durchmesser des darauf getrockneten und verteilten Gutes für die nachfolgende Untersuchung zu desorbieren, um für die Analyse bzw. Diagnostik eine definiertere Blutmenge bereitstellen zu können. Die so gewonnene Scheibe wird mit einem geeigneten Lösemittel extrahiert, der Extrakt zentrifugiert und der Überstand aufgereinigt oder unmittelbar bzw. nach einem Austausch des Lösemittels vermessen.

Durch die WO 2015/044454 A2 ist es zur Verbesserung der Auswerte- bzw. Messergebnisse bekannt geworden, die aus einem Probenträgerpapier, insbesondere durch Ausstanzen, zu desorbierende Menge getrockneten Bluts durch Kapillarkanäle exakter zu bestimmen. Dazu wird das unbestimmt entnommene Flüssigkeitsvolumen in mehrere, jeweils eine bestimmte Blutmenge aufnehmende Kapillarkanäle eingeleitet, nach deren völliger Befüllung der Zufluss an der Eingabestelle abreißt, so dass in der Folge am Auslaufende eines jeden Kapillarkanals nur exakt diese durch das Aufnahmevolumen des Kapillarkanals bestimmte bzw. kalibrierte Blutsmenge in einen für die von jedem Kapillarkanal bestimmte Blutsmenge zur Ausstanzung vorgesehenen Bereich mit einem Durchmesser von etwa 6 mm auf das Probenträgerpapier bzw. eine Probenträgersammelkarte gelangt. Damit die kalibrierte Blutsmenge auf die Probenträgersammelkarte übergeleitet werden kann, ist am Auslaufende der Kapillarkanäle eine lösliche, nach dem Auflösen den Durchfluss erlaubende Membrane ausgebildet.

Weitere Vorrichtungen zur Aufnahme von Flüssigkeitsproben sind aus der WO 2015/044454 A2, der US 2013/280725 A1, der US 2013/281316 A1, der WO 2015/058958 A1, der US 2004/126281 A1, der WO 2013/148071 A1, der US 2001/039057 A1 und der US 4 883 764 A bekannt.

Der Erfindung liegt die Aufgabe zugrunde, eine bekannte Probenträger-Bereitstellungsschachtel für eine alternative Entnahme der Probenträger weiterzubilden. Diese Aufgabe wird mit dem Gegenstand des unabhängigen Patentanspruchs 1 gelöst.

Beansprucht wird eine Probenträger-Bereitstellungsschachtel, bei der im Abstand voneinander angeordnete, eine bestimmte, festgelegte Flüssigkeit-Teilmenge durch Kapillarzug aufsaugende Probenträger zwischen einer Systemplatte und einem Gehäuse eingeschlossen sind, wobei das Gehäuse mit einem domartigen Einfüllstutzen versehen ist, der über der Systemplatte auf in diese ausgebildeten, sich zu den Probenträgern verzweigenden Kapillarkanälen endet, und wobei die in der Probenträger-Bereitstellungsschachtel getrockneten Probenträger durch eine Relativbewegung von Systemplatte und Gehäuse eine Position oberhalb von Entnahmeöffnungen einnehmen, durch die Probenträger nach unten aus der Bereitstellungsschachtel herausfallen können. Die in ihren Abmessungen kleingehaltene Probenträger-Bereitstellungsschachtel kammert die gesamte eingefüllte Flüssigkeitsmenge zur und während der Blutverteilung bis einschließlich der Trocknung ein. Denn auch der Einfüllstutzen, der zur Aufnahme überschüssiger Flüssigkeit ein Aufnahmevolumen haben kann, das geringfügig größer als das aller saugfähigen Probenkörper ist, kann durch einen Stopfen verschlossen werden. Der sich verzweigende Kapillarkanal bzw. die damit vorliegenden Kapillarkanäle, der bzw. die im Zusammenspiel mit den saugfähigen Probenkörpern ausschließlich der Kapillarwirkung und nicht wie beim Stand der Technik der Kalibrierung der Flüssigkeitsmenge dienen, können durch eine Heißversiegelung oder eine Abdeckung in ihrer Kapillarwirkung unterstützt werden.

Die beanspruchte Probenträger-Bereitstellungsschachtel funktioniert wie folgt:
Bei ihr wird die aufgegebene unbestimmte Flüssigkeitsmenge in einem geschlossenen System durch Kapillarwirkung mit genauer Aufteilung an eine Mehrzahl von eine bestimmte Flüssigkeits-Teilmenge aufsaugenden Probenträgern gezielt zugeführt, wobei die Probenträger nach dem Trocknen der Flüssigkeits-Teilmengen zur Analyse über einen temporär nur zur Abgabe der Probenträger und auf diesen begrenzten, geöffneten Bereich aus dem geschlossenen System einzeln entnommen werden können. Indem nicht mehr die Kapillarkanäle, sondern die Probenträger selbst die Führungsgröße zur Bestimmung der jeweiligen, exakten Teilmengen aus der unbestimmt aufgegebenen Flüssigkeitsmenge sind, lässt sich vermeiden, dass das System schon vor der Trocknung der bestimmten Flüssigkeits- bzw. Blutmenge aufgelöst werden muss. Das geschlossene System verhindert daher mit größter Sicherheit, dass flüssiges Blut nach außen dringen kann. Dieses wird vielmehr nur intern durch die Kapillarwirkung gezielt an die Probenträger weitergeleitet und von diesen in einer auf ein vorher festgelegtes Maß bestimmten Teilmenge der Gesamtflüssigkeit aufgenommen. Erst wenn die Teilmenge getrocknet ist, wird das System partiell geöffnet, und zwar dort, wo ein Probenträger ausgeworfen werden soll. Ein Ausstanzvorgang entfällt dabei völlig, denn die Probenträger liegen in dem geschlossenen System schon vereinzelt vor.

Hierzu eignen sich Probenkörper, die aus verpressten Kunststoffkugeln bestehen, deren natürliche Oberflächenbeschaffenheit eine Polarität besitzt, die eine kapillarfördernde Wirkung hat. Optional lassen sich die Kunststoffkugeln durch eine Oberflächenbehandlung in einen saugfähigen Zustand versetzen. Das Aufnahmevolumen der Probenträger lässt sich dabei durch die Zwischenräume der einzelnen Kugeln bestimmen, so dass Variationen durch die Wahl der geometrischen Abmessungen möglich sind. Nach der Füllung der saugfähigen Probenkörper mit der durch diese bestimmten Flüssigkeits-Teilmenge ist kein weiterer Kapillarzug mehr vorhanden und ein Überschuss der aufgegebenen Flüssigkeitsmenge verbleibt außerhalb der Probenkörper in dem geschlossenen System. Dieses wird nur zur Entnahme der Probenkörper nach der Trocknung der Flüssigkeit in einem zur Entnahme der Probenkörper begrenzten Bereich geöffnet.

Bei einer ersten erfindungsgemäßen Ausführung einer Probenträger-Bereitstellungsschachtel sind Systemplatte und Gehäuse viereckig, wobei die in das Gehäuse schubladenartig eingeschobene Systemplatte in ihrem in Einschubrichtung vorderen Endbereich mehrere in einer Reihe angeordnete Kammern für die Probenträger aufweist und mit einer ihren hinteren Endbereich im Auslieferungs- und Befüllzustand an der Einschubseite der Probenträger-Bereitstellungsschachtel hervorstehend haltenden Raststufe ausgebildet ist, die durch die Einschubkraft überwunden werden kann, wobei in der völlig eingeschobenen Endposition der Systemplatte die Kammern mit den Probenträgern eine zu den Entnahmeöffnungen des Gehäuses fluchtende Lage einnehmen. Die aus dem Gehäuse hervorstehende Lage der Systemplatte kann nach einer Ausgestaltung der Erfindung durch in den Seitenwänden des hervorstehenden hinteren Endbereiches der Systemplatte zusätzlich vorgesehene, nach außen ausgestellte Einschubsicherungslaschen unterstützt werden. Die Einschubsicherungslaschen legen sich dabei an die freien Stirnkanten der Gehäusewand an.

Durch die vorstehenden Maßnahmen lässt sich gewährleisten, dass die Probenträger während der Befüllung und Trocknung wirksam vollumfänglich eingeschlossen sind und keine Flüssigkeit bzw. Blut nach außen dringen kann.

Nach einem vorteilhaften Vorschlag der Erfindung ist das Gehäuse mit einer in der Endposition der eingeschobenen Systemplatte die Probenträger aus ihrem Sitz in den Kammern lösenden und in die Entnahmeöffnungen ausstoßenden Anschlagrampe ausgebildet. Durch Zusammendrücken der beiden gegenüberliegenden Einschubsicherungslaschen mit zwei Fingern einer Hand kann bei gleichzeitiger Überwindung der Raststufe die Systemplatte in das Gehäuse bis in ihre Endlage eingeschoben werden. Dabei trifft die Systemplatte kurz vor ihrer Endlage auf die Anschlagrampe, die im Zuge des weiteren Einschiebens bis zur Endlage die Probenträger löst und über die Entnahmeöffnungen nach unten ausstößt, vorzugsweise sogleich in eine weiterverarbeitende Einheit, auf die die Probenträger-Bereitstellungsschachtel zuvor zentriert aufgesetzt worden ist.

Eine andere Ausführung einer Probenträger-Bereitstellungsschachtel sieht vor, dass Systemplatte und Gehäuse rund sind, wobei das Gehäuse mehrere auf einem Innenkreis angeordnete Kammern für die Probenträger aufweist und auf der Systemplatte verdrehbar verrastet ist, wobei die Systemplatte mit den Entnahmeöffnungen versehen ist, die zur Abgabe von Probenträgern durch eine Drehbewegung des Gehäuses bis in eine zu den Kammern fluchtende Lage gebracht werden können. Auch hierbei wird das geschlossene System erst zur Entnahme der getrockneten Probenkörper bereichsweise geöffnet, nämlich in der durch einen Anschlag festgelegten Drehposition des Gehäuses, in der die Probenkörper mit den Entnahmeöffnungen fluchten, vorzugsweise ebenfalls mit Übergang direkt in eine weiterverarbeitende Einheit.

Nach einer Ausgestaltung der Erfindung sind deshalb die Entnahmeöffnungen des Gehäuses bzw. die bei der runden Ausführung in der Systemplatte ausgebildeten Entnahmeöffnungen mit einem die Probenträger-Bereitstellungsschachtel beim Aufsetzen auf eine weiterverarbeitende Einheit zentrierenden Außenkragen versehen.

Die runde Probenträger-Bereitstellungsschachtel kann zum Ausstoßen bzw. Entnehmen der getrockneten Probenträger oberhalb der Kammern mit den Probenträgern angeordnete Ausdrücknoppen aufweisen. Statt am Gehäuse stationäre Ausdrücknoppen vorzusehen, können diese nach einem anderen Vorschlag der Erfindung an einem über ein elastisches Gelenk mit der Systemplatte befestigten Schwenkdeckel vorgesehen werden.

Es wird vorgeschlagen, dass vorteilhaft zumindest das Gehäuse aus einem transparenten Material besteht. Die Transparenz ermöglicht in einfacher Weise eine optische Kontrolle der Verteilung der aufgegebenen Flüssigkeit auf die Probenträger. Es liegt dabei im Rahmen der Gegebenheiten, dass das Gehäuse mit einer Beschriftung, mit einem Etikett oder mit einem Barcode versehen werden kann.

Eine weitere Ausführung eines erfindungsgemäß geschlossenen Systems mit Flüssigkeitsverteilung durch Kapillarwirkung und Probenträgern, die eine exakt bestimmte Flüssigkeits-Teilmenge aufnehmen, kann darin bestehen, dass die einzelnen Probenträger über- bzw. hintereinander in einem zylindrischen, röhrchenförmigen Gehäuse bereit gestellt werden, das sich zentriert auf ein weiterverarbeitendes System aufsetzen lässt und anschließend die aufeinander folgende Abgabe einzelner Probenträger ermöglicht. Dies lässt sich durch Niederdrücken eines an der von der Entnahmeöffnung des zylindrischen Gehäuses gegenüberliegenden Seite in das Gehäuse eintauchenden Stößels erreichen, der die einzelnen Probenträger haltende, flexible Gehäusearme trennt. Die offene Entnahmeseite, die gleichzeitig zur Befüllung mit dem Blut dient, kann durch einen Stopfen verschlossen werden. Wenn nach dem Einfüllen des Blutes das zylindrische Gehäuse mit der Entnahmeöffnung nach oben weisend in eine senkrechte Position gestellt wird, reicht gegebenenfalls allein die Schwerkraft zusammen mit der Kapillarwirkung der saugfähigen Probenkörper für die Verteilung der aufgegebenen Blutmenge auf die einzelnen Probenkörper aus.

Weitere Merkmale und Einzelheiten der Erfindung ergeben sich aus den Ansprüchen und der nachfolgenden Beschreibung von in den Zeichnungen dargestellten Ausführungsbeispielen der Erfindung. Es zeigen:
- Figur 1: als Schemaskizze in der Draufsicht am Beispiel einer Probenträger-Bereitstellungsschachtel nach den Figuren 2 bis 8 die Aufteilung eines eingefüllten, unbestimmten Flüssigkeitsvolumens durch Kapillarwirkung in einem geschlossenen System in mehrere exakt definierte Mengen;
- Figur 2: in einer perspektivischen Draufsicht eine in viereckiger Flachbauweise ausgebildete Probenträger-Bereitstellungsschachtel im Auslieferungszustand, in dem eine schubladenartig in ein äußeres Gehäuse einschiebbare Systemplatte durch eine Raststufe mit ihrem in Einschubrichtung hinteren Bereich aus dem Gehäuse vorkragend gehalten wird;
- Figur 3: eine Darstellung wie in Figur 2, demgegenüber ohne den einen zentralen, domartigen Einfüllstutzen verschließenden Stopfen;
- Figur 4: in einer perspektivischen Ansicht wie zuvor die zur Entnahme von Probenträgern mit getrocknetem Blut völlig in das Gehäuse eingeschobene Systemplatte, gegenüber den Figuren 2 und 3 um 180° gedreht, d. h. von unten gesehen dargestellt;
- Figur 5: in vergrößerter Darstellung als Einzelheit den in den Figuren 4 und 8 strichpunktiert eingekreisten, die Funktionslage zum Entnehmen der Probenträger wiedergebenden Bereich, in dem bei in der Endlage verschobener Systemplatte die Probenträger bzw. deren sie aufnehmenden Kammern in Flucht mit Entnahmeöffnungen des Gehäuses verlaufen;
- Figur 6: die Probenträger-Bereitstellungsschachtel der Figur 3 als Gesamtansicht in einer anderen Perspektive von oben, der Einfüllseite her gesehen mit an der Bruchlinie endender Gehäuse-Oberfläche;
- Figur 7: einen Schnitt entlang der Linie VII-VII von Figur 2;
- Figur 8: einen Querschnitt wie zuvor in Figur 7, demgegenüber mit der zur Entnahme der Probenträger in ihre Endlage eingeschobenen Systemplatte (vergl. hier Figur 4);
- Figur 9: in einer perspektivischen Draufsicht eine in runder Form ausgebildete Probenträger-Bereitstellungsschachtel;
- Figur 10: einen Schnitt entlang der Linie X-X von Figur 9;
- Figur 11: einen Querschnitt wie zuvor in Figur 10, demgegenüber das auf der Systemplatte in die Abgabeposition zur Entnahme der Probenträger verdrehte Gehäuse;
- Figur 12: eine perspektivische Draufsicht wie in Figur 9, demgegenüber mit einer durch einen Schwenkdeckel abgewandelten Ausführung zum Ausdrücken der in die Abgabeposition bewegten Probenträger; und
- Figur 13: in einem Querschnitt der Figur 12 die Abgabeposition zur Entnahme der Probenträger.

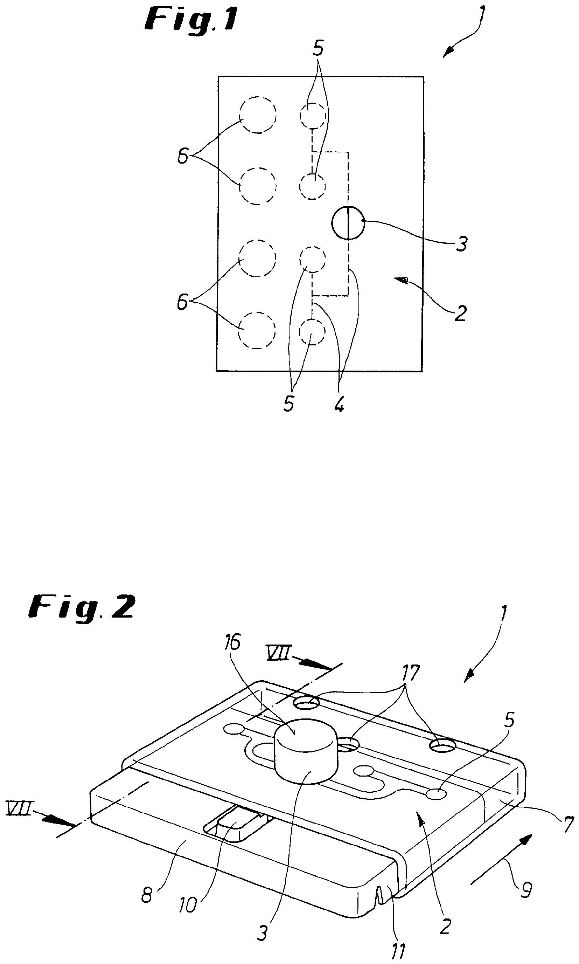

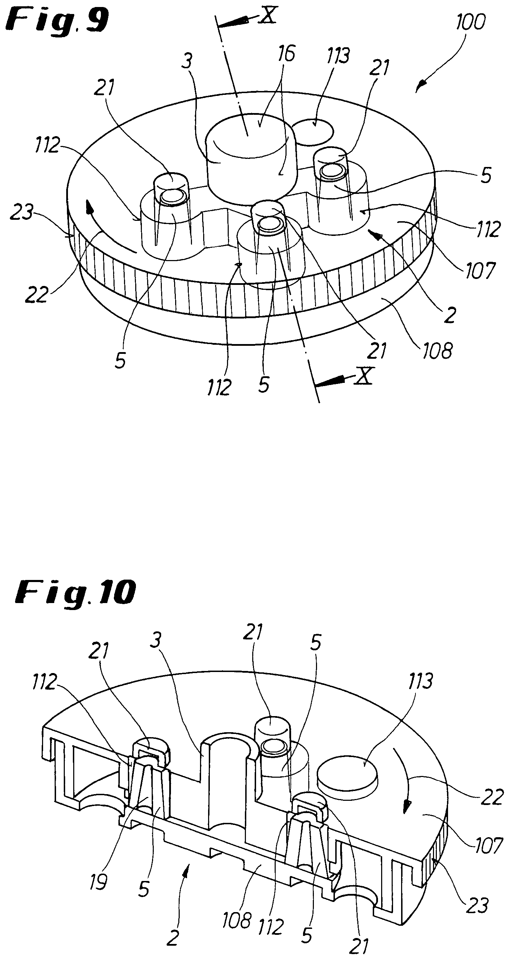

Die Figur 1 zeigt das mit Probenträger-Bereitstellungsschachteln 1 (vergl. die Figuren 2 bis 8) und 100 (vergl. die Figuren 9 bis 13) grundsätzlich realisierte Prinzip, demgemäß ein unbestimmtes Flüssigkeitsvolumen durch Kapillarwirkung in einem geschlossenen System 2 in mehrere exakt definierte Mengen aufgeteilt, getrocknet und wieder abgeben werden kann, wobei die Anwender eine große Sicherheit gegenüber Berührung der Flüssigkeit, insbesondere Blut, besitzen. Es ist daher z. B. die Blutabnahme durch die Patienten selbst zuhause möglich, mit dem anschließenden Versand der Proben zur Analyse. Über eine bei den Probenträger-Bereitstellungsschachteln 1, 100 konkret als Einfüllstutzen 3 ausgebildete Einfüllöffnung wird die unbestimmte Blutmenge aufgegeben und über einen sich in Einzelkanäle verzweigenden Kapillarkanal 4 zu den saugfähigen Probenträgern 5, die eine von vornherein bestimmte bzw. genau dosierte Blutmenge aufnehmen können, gezielt weitergeleitet. Nach dem Trocknen der Flüssigkeit- bzw. Blut-Teilmenge können die Probenträger 5 aus ihrer in dem geschlossenen System 2 eingekammerten Lage in eine Entnahmeposition bewegt werden, in der sie sich fluchtend über nach unten offenen Entnahmeöffnungen 6 befinden.

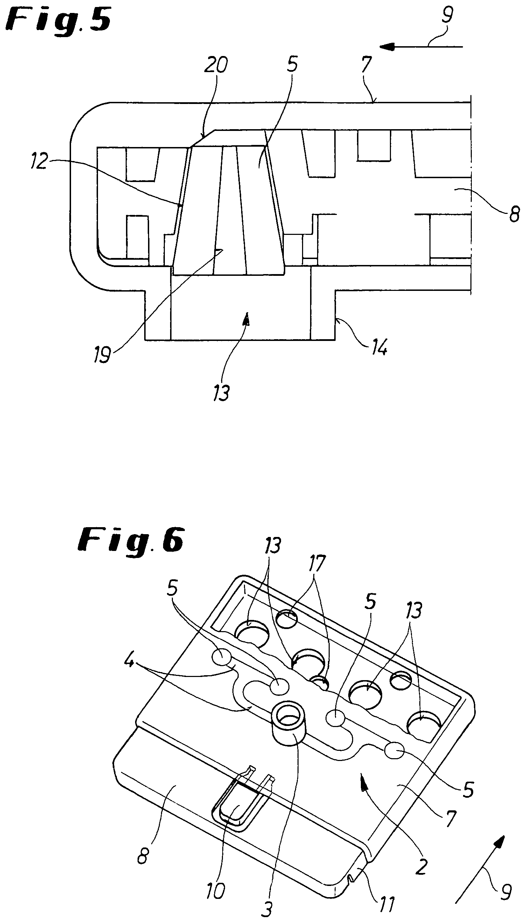

Die in den Figuren 2 bis 8 in einer ersten Ausführung dargestellte Probenträger-Bereitstellungsschachtel 1 besitzt ein rechteckiges Format und besteht aus einem flachen, einseitig offenen Gehäuse 7, in das eine den sich verzweigenden Kapillarkanal 4 (vergl. Figur 6) aufweisende Systemplatte 8 einschiebbar ist. Den Figuren 2, 3 und 6 lässt sich der Auslieferungszustand der Probenträger-Bereitstellungsschachtel 1 entnehmen. Die Systemplatte 8 ragt mit ihrem in Einschubrichtung 9 hinteren Bereich etwas aus der offenen Einschubseite des Gehäuses 7 nach außen hervor. Diese Lage wird durch eine nach oben gegen die Oberwand des Gehäuses 7 vorstehende Raststufe 10 in Form einer gefedert angelenkten Rastnase (vergl. Figur 6) gesichert. Zum Schutz gegen unbeabsichtigtes Einschieben sind in den beiden Seitenwänden der Systemplatte 8 zusätzlich nach außen ausgestellte, beim Einschieben nach innen ausweichende Einschubsicherungslaschen 11 vorgesehen.

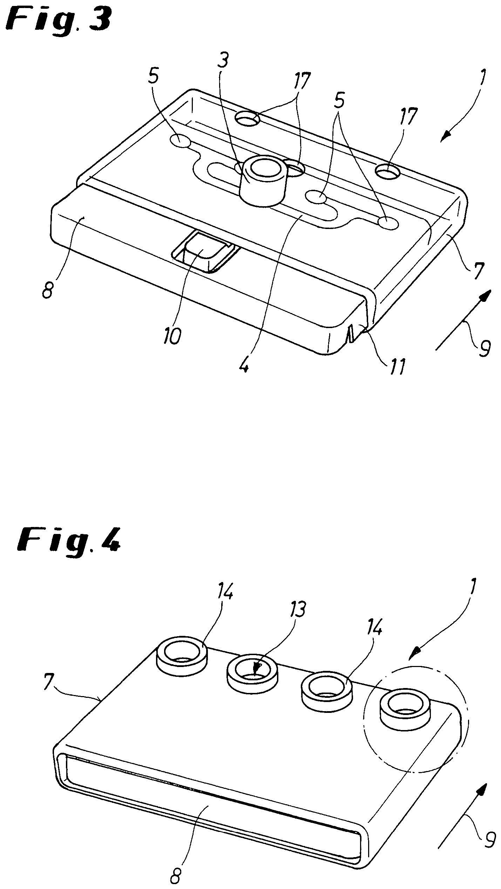

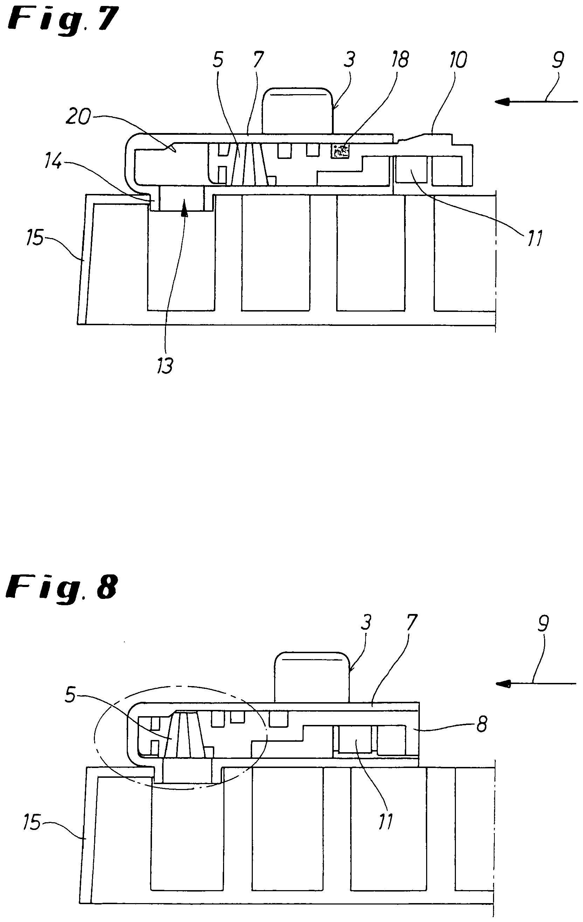

In der Probenträger-Bereitstellungsschachtel 1 sind in dem in Einschubrichtung 9 vorderen Bereich der Systemplatte 8 in Reihe vier voneinander beabstandete, umfangsgeschlossene Kammern 12 (vergl. Figur 7) zur Aufnahme von somit vier saugfähigen Probenträgern 5 (vergl. Figur 6) angeordnet. Im Auslieferungszustand, d. h. vorbereitet zur Befüllung mit entnommenem Blut, sind die Kammern 12 völlig geschlossen, nämlich durch die Oberwand und Bodenwand des Gehäuses 7 abgedeckt, wie aus der Figur 7 zu sehen ist. In dem in Einschubrichtung 9 vorderen Bereich der Probenträger-Bereitstellungsschachtel 1 bzw. in einem von der Einschubseite her gesehen hinteren Endabschnitt des Gehäuses 9 sind in deren Bodenwand entsprechend den Kammern 12 der Probenträger 5 in Reihe voneinander beabstandet vier Entnahmeöffnungen 13 vorgesehen (vergl. Figur 6), die an der Unterseite des Gehäuses 7 von Außenkragen 14 eingefasst sind (vergl. Figur 4). Mittels der Außenkragen 14 lässt sich die Probenträger-Bereitstellungsschachtel 1 zur Abgabe der getrockneten und ausgestoßenen Probenträger 5 auf eine weiterverarbeitende Einheit 15 aufsetzen, wie in den Figuren 7 und 8 gezeigt.

Die durch einen Stopfen 16 (vergl. Figur 2) verschließbare, als domartiger Einfüllstutzen 3 ausgebildete Einfüllöffnung ist zentral auf der Oberwand des Gehäuses 7 vorgesehen und mündet über den sich zu den Probenträgern 5 verzweigenden Kapillarkanal 4 der Systemplatte 8. Das Aufnahmevolumen des domartigen Einfüllstutzens 3 ist geringfügig größer als das Aufnahmevolumen aller saugfähigen Probenträger 5 zusammen, die ausgelegt werden können, 3 bis 200 µ I aufzunehmen, was dem Anwender eine grobe Abschätzung des insgesamt benötigten Probevolumens ermöglicht. Nachdem sich die Probenträger 5 mit dem bestimmten bzw. vorbestimmten Volumen gefüllt haben, liegt in dem sich verzweigenden Kapillarkanal 4 kein Kapillarzug mehr vor und die überschüssige Flüssigkeit bzw. Blutmenge verbleibt in dem Kapillarkanal. Wenn das Gehäuse 7 vorteilhaft aus einem transparenten Kunststoffmaterial besteht, kann die ordnungsgemäße Probenverteilung vom Anwender optisch kontrolliert bzw. überwacht werden.

Die Trocknung der von den Probenträgern 5 aufgenommenen Flüssigkeit wird durch Belüftung des geschlossenen Systems über einerseits den Einfüllstutzen 3 und andererseits die Entnahmeöffnungen 13 begünstigt. Als die Trocknung unterstützende Maßnahmen kann die Systemplatte 8 und / oder das Gehäuse 7 mit Konvektionsöffnungen 17, wie aus den Figuren 2 und 3 beispielsweise zu erkennen, versehen werden, dem Kapillarkanal 4 können trocknungsunterstützende Mittel zugegeben werden, z. B. wie in Figur 7 angedeutet Granulat 18, und die Probenträger 5 können ein axiales Durchgangsloch 19 aufweisen.

Die Probenträger-Bereitstellungsschachtel 1 mit den exakt proportionierte Blutmengen aufweisenden Probenträgern 5 kann nach der Trocknung im Ausgangszustand der Figuren 2 und 3 zur Analyse über die Außenkragen 14 der Entnahmeöffnungen 13 zentriert auf eine weiterverarbeitende Einheit 15 aufgesetzt werden (vergl. Figur 7). Sobald die Systemplatte 8 dann in ihre in Figur 4 dargestellte Endlage eingeschoben wird, verlagern sich die Probenträger 5 aus der in Figur 7 gezeigten Bereitstellungsposition in die in Figur 8 dargestellte Entnahmeposition, in der sie sich fluchtend über den Entnahmeöffnungen 13 des Gehäuses 7 befinden und nach unten in die weiterverarbeitende Einheit 15 ausgestoßen werden können. Damit das in einem Bewegungsablauf beim Einschieben der Systemplatte 8 erfolgen kann, ist die Oberwand des Gehäuses 7 im Bereich der Endlagenposition mit einer Rampe 20 ausgebildet, die beim Auftreffen der Probenträger 5 deren selbsttätiges Ablösen aus den Kammern 12 und Herunterfallen in die weiterverarbeitende Einheit 15 ermöglicht.

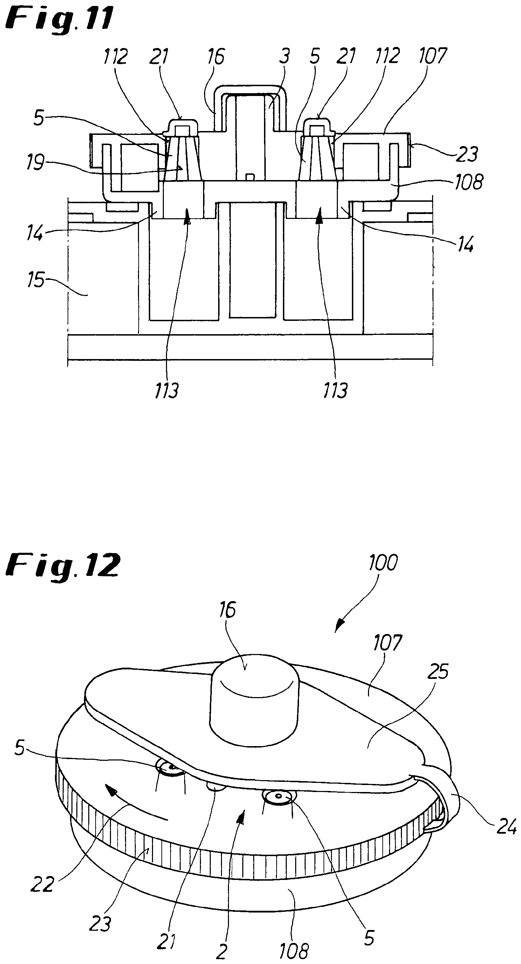

Bei einer in den Figuren 9 bis 13 dargestellten Variante zur kapillaren Aufteilung eines unbestimmten Flüssigkeitsvolumens in mehrere exakt definierte Teilmengen in einem geschlossenen System unterscheidet sich im Wesentlichen allein die Form der Probenträger-Bereitstellungsschachtel 100, die hier nämlich rund ist, von dem zuvor beschriebenen Funktionsprinzip. Denn unverändert wird über den nur zum Einfüllen der entnommenen Flüssigkeitsmenge bzw. des Blutes ohne Stopfen 16 frei zugänglichen, domartigen Einfüllstutzen 3 das unbestimmte Flüssigkeitsvolumen eingefüllt, das sich in der Folge über den sich verzweigenden Kapillarkanal der Systemplatte 108 zu den auf einem Innenkreis im Karree angeordneten Probenträgern 5 verteilt. In diesem Fall sind die die Probenträger 5 eingehaust aufnehmenden Kammern 112 an dem transparenten, runden Gehäuse 107 und die Entnahmeöffnungen 113 an der Systemplatte 108 ausgebildet (vergl. die Figuren 10 und 11).

Die Figur 9 zeigt den Auslieferungszustand der Probenträger-Bereitstellungsschachtel 100, in der der Einfüllstutzen 3 durch den aufgesetzten Stopfen 16 verschlossen wird. Zum Gebrauch bzw. beim Befüllen mit einer entnommenen unbestimmten Blutmenge wird der Stopfen 16 entfernt, wie in Figur 10 gezeigt, wobei die Probenkörper 5 bei ansonsten völlig geschlossenem System 2, nämlich einerseits mit unterer Abdeckung der Kammern 112 durch die Systemplatte 108 und andererseits oben von an dem Gehäuse 107 vorgesehenen, den Kammern 112 zugeordneten Ausdrücknoppen 21, durch Kapillarzug die aufgeteilten, bestimmten Flüssigkeits-Teilmengen ansaugen bzw. aufnehmen. Zum Versand wird der Einfüllstutzen 3 der Probenträger-Bereitstellungsschachtel 100 durch den Stopfen 16 bis einschließlich der Entnahme der Probenträger 5 zur Analyse wieder verschlossen.

Um die Probenträger 5 zu analysieren, wird die Probenträger-Bereitstellungsschachtel 100, wie in Figur 11 gezeigt, über die Außenkragen 14 der Entnahmeöffnungen 113 der Systemplatte 108 zentriert auf die weiterverarbeitende Einheit 15 aufgesetzt. Anschließend werden die Kammern 112 mit den Probenträgern 5 durch eine Drehbewegung (vergl. den Drehbewegungspfeil 22 in den Figuren 9 und 10) des transparenten Gehäuses 107 in die in Figur 11 dargestellte, durch einen Rastanschlag festgelegte, mit den Entnahmeöffnungen 113 der Systemplatte 108 fluchtende Lage gebracht. Die Probenträger 5 können danach durch Niederdrücken der Ausdrücknoppen 21 in die weiterverarbeitende Einheit 15 ausgeworfen werden. Zur Griffsicherheit beim Verdrehen des Gehäuses 107 gegenüber der Systemplatte 108 ist der die Systemplatte 108 nach unten etwas überlappende Außenrand des Gehäuses 107 mit einer geriffelten Grifffläche 23 versehen.

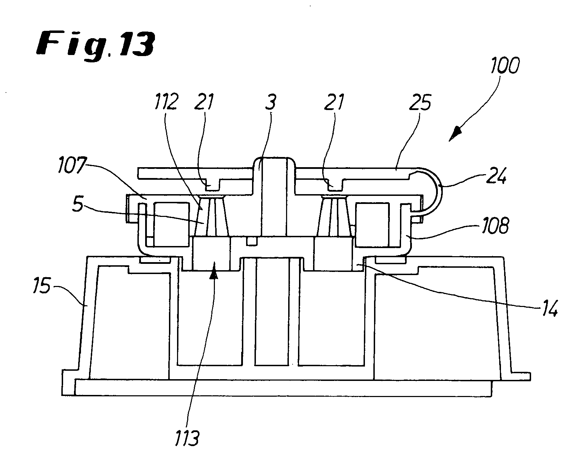

Bei der in den Figuren 12 (Auslieferungszustand) und Figur 13 (Probenträger-Auswurfsituation zur Analyse) dargestellten Abwandlung der runden Probenträger-Bereitstellungsschachtel 100 sind die Ausdrücknoppen 21 an einem über ein elastisches Gelenk 24 mit der Systemplatte 108 befestigten Schwenkdeckel 25 so positioniert angeordnet, dass sie nach dem Verdrehen des Gehäuses 107 über den Kammern 112 mit den Probenträgern 5 liegen (vergl. Figur 13) und durch Niederdrücken die Probenträger über die Entnahmeöffnungen 113 in die durch Außenkragen 14 zentriert darunter befindliche weiterverarbeitende Einheit 15 ausgestoßen werden können.

### Bezugszeichenliste:

- 1, 100: Probenträger-Bereitstellungsschachtel
- 2: geschlossenes System
- 3: Einfüllstutzen / Einfüllöffnung
- 4: Kapillarkanal
- 5: Probenträger
- 6: Entnahmeöffnung
- 7, 107: Gehäuse
- 8, 108: Systemplatte
- 9: Einschubrichtung
- 10: Raststufe / Rastnase
- 11: Einschubsicherungslasche
- 12, 112: Kammer
- 13, 113: Entnahmeöffnung
- 14: Außenkragen
- 15: weiterverarbeitende Einheit
- 16: Stopfen
- 17: Konvektionsöffnung
- 18: Granulat
- 19: Durchgangsloch
- 20: Rampe / Anschlagrampe
- 21: Ausdrücknoppen
- 22: Drehbewegungspfeil
- 23: Grifffläche
- 24: elastisches Gelenk
- 25: Schwenkdeckel

## Patentansprüche

1. Probenträger-Bereitstellungsschachtel (1, 100) zum Bereitstellen von einer getrockneten Flüssigkeitsmenge, insbesondere Blut, aufweisenden Probeträgern,
▪ mit einem Gehäuse (7,107) und einer relativ zum Gehäuse beweglichen Systemplatte (8,108),
• mit einer auf dem Gehäuse (7,107) als domartigen Einfüllstutzen (3) ausgebildeten Einfüllöffnung,
• mit einem Kapillarkanal (4), der in der Systemplatte ausgebildet ist,
• mit einer Mehrzahl von Kammern (12) in der Systemplatte (8, 108), die Kammern (12) aufweisend je einen saugfähigen Probenträger (5) zur Aufnahme einer vorbestimmten Blutmenge,
• wobei der Kapillarkanal (4) mit der Einfüllöffnung verbunden ist und sich in mehrere Einzelkanäle verzweigt, die zu den Kammern (12) mit den saugfähigen Probenträgern (5) führen,
• wobei der mit einem Stopfen verschließbare Einfüllstutzen (3) und der Kapillarkanal mit den Kammern (12) und Probenträgern (5) ein geschlossenes System (2) bilden,
• wobei, indem der Kapillarkanal (4) mit der Einfüllöffnung verbunden ist und sich in mehrere Einzelkanäle verzweigt, die zu den Kammern (12) mit den saugfähigen Probenträgern (5) führen, durch Kapillarwirkung eine über die Einfüllöffnung aufgegebene unbestimmte Flüssigkeitsmenge in dem geschlossenen System (2) zu den saugfähigen Probenträgern (5) weitergeleitet werden kann,
**dadurch gekennzeichnet, dass**
die Probenträger (5) nach dem Trocknen der von ihnen aufgenommenen vorbestimmten Flüssigkeits-Teilmenge durch eine Relativbewegung von Systemplatte (7, 107) und Gehäuse (8, 108) aus ihrer in dem geschlossenen System (2) eingekammerten Lage in eine Entnahmeposition bewegbar sind, in welcher sie sich fluchtend über nach unten offenen Entnahmeöffnungen der Probenträger-Bereitstellungsschachtel (1, 100) befinden.

## Claims

1. A sample carrier preparation package (1, 100) for the preparation of sample carriers having a dried quantity of liquid, in particular blood,
▪ with a housing (7, 107) and a system panel (8, 108), which moves relative to the housing,
• with a filling opening configured as a dome-like filling orifice (3),
• with a capillary channel (4) which is formed in the system panel,
• with a plurality of chambers (12) in the system panel (8, 108), the chambers (12) each having an absorbent sample carrier (5) for receiving a predetermined quantity of blood,
• wherein the capillary channel (4) is connected to the filling opening and branches into a plurality of individual channels which lead to the chambers (12) with the absorbent sample carriers (5),
• wherein the filling orifice (3) which can be closed by a stopper and the capillary channel with the chambers (12) and sample carriers (5) form a closed system (2),
• wherein, because it is connected to the filling opening and branches into a plurality of individual channels which lead to the chambers (12) with the absorbent sample carriers (5), in the closed system (2), the capillary channel (4) can transfer an indeterminate quantity of liquid applied via the filling opening to the absorbent sample carriers (5) by capillary action,
**characterized in that**
after drying the predetermined part-quantity of liquid received thereby, the sample carriers (5) can be moved by means of a relative movement of the system panel (7, 107) and housing (8, 108) from their position enclosed in the closed system (2) into a removal position in which they are aligned over removal openings of the sample carrier preparation package (1, 100) which are open at the bottom.

## Revendications

1. Boîte de fourniture de supports d'éprouvettes (1, 100) pour fournir des supports d'éprouvette présentant une quantité de liquide séché, en particulier du sang,
▪ comprenant un logement (7, 107) et une plaque de système (8, 108) mobile par rapport au logement,
• comprenant une ouverture de remplissage formée en tant que tubulure de remplissage (3) en forme de dôme sur le logement (7, 107),
• comprenant un canal capillaire (4) qui est formé dans la plaque de système,
• comprenant une pluralité de chambres (12) dans la plaque de système (8, 108), les chambres (12) présentant respectivement un support d'éprouvette absorbant (5) pour recevoir une quantité de sang prédéterminée,
• dans lequel le canal capillaire (4) est relié à l'ouverture de remplissage et s'embranche en plusieurs canaux individuels qui conduisent aux chambres (12) avec les supports d'éprouvette absorbants (5),
• dans lequel la tubulure de remplissage (3) pouvant être fermée avec un bouchon et le canal capillaire avec les chambres (12) et les supports d'éprouvette (5) forment un système fermé (2),
• dans lequel, par le fait que le canal capillaire (4) soit relié à l'ouverture de remplissage et s'embranche en plusieurs canaux individuels qui conduisent aux chambres (12) avec les supports d'éprouvette absorbants (5), une quantité de liquide indéfinie dans le système fermé (2), sortie par capillarité par l'ouverture de remplissage, peut être conduite vers les supports d'éprouvette absorbants (5),
**caractérisé en ce que**
les supports d'éprouvette (5), après le séchage de la quantité partielle de liquide prédéterminée qu'ils ont reçue, sont mobiles par un mouvement relatif de la plaque de système (7, 107) et du logement (8, 108) de leur position enfermée dans le système fermé (2) à une position de prélèvement, dans laquelle ils se trouvent alignés au-dessus d'ouvertures de prélèvement de la boîte de fourniture de supports d'éprouvettes (1, 100) ouvrant vers le bas.
